# EUROPEAN PATENT SPECIFICATION

(11) **EP 3 413 785 B1**
(45) Date of publication and mention of the grant of the patent: **27.10.2021**
(21) Application number: 17706374.0
(22) Date of filing: 10.02.2017
(51) Int. Cl.: A61B 5/00, A61B 5/1459, A61B 17/00, A61B 5/107, A61B 5/026

(54) **SURGICAL SYSTEM**
CHIRURGISCHES SYSTEM
SYSTÈME CHIRURGICAL

(30) Priority: 13.02.2016 US 201662295005 P
(43) Date of publication of application: 19.12.2018
(73) Proprietor: Briteseed, LLC, Chicago, IL 60640 (US)
(72) Inventor: GUNN, Jonathan, Chicago IL 60611 (US); CORRIGAN, Sean, Chicago IL 60647 (US); GREENE, Daniel, Joseph, Chicago IL 60640 (US); LEATZOW, Derek, J., Chicago IL 60613 (US); PAPADOPOULOS, Marcur, Stephen, Paradise Valley AZ 85253 (US); LE ROLLAND, Paul, Chicago IL 60614 (US); MATUSAITIS, Tomas, Chicago IL 60625 (US)
(74) Representative: J A Kemp LLP
(86) International application number: PCT/US2017/017468
(87) International publication number: WO 2017/139642

(56) References cited:
- WO-A1-2004/030527
- WO-A1-2015/148504
- GB-A- 1 445 678
- US-A- 5 991 650
- US-A1- 2006 052 850
- US-A1- 2007 219 551
- US-A1- 2012 172 842
- US-A1- 2014 187 874

## Description

### Background

This patent is directed to a system for electrically coupling a surgical system or part thereof, and in particular to a system for electrically coupling at least one light emitter and at least one light sensor disposed at a distal end of a tubular shaft to the remainder of the surgical system.

Systems and methods that identify artifacts, and in particular vessels, in the surgical field during a surgical procedure provide valuable information to the surgeon or surgical team. U.S. hospitals lose billions of dollars annually in unreimbursable costs because of inadvertent vascular damage during surgery. In addition, the involved patients face a mortality rate of up to 32%, and likely will require corrective procedures and remain in the hospital for an additional nine days, resulting in tens, if not hundreds, of thousands of dollars in added costs of care. Consequently, there is this significant value to be obtained from methods and systems that permit accurate determination of the presence of vessels, such as blood vessels, in the surgical field, such that these costs may be reduced or avoided.

Systems and methods that provide information regarding the presence of blood vessels in the surgical field are particularly important during minimally-invasive surgical procedures. Traditionally, surgeons have relied upon tactile sensation during surgical procedures both to identify blood vessels and to avoid inadvertent damage to these vessels. Because of the shift towards minimally-invasive procedures, including laparoscopic and robotic surgeries, surgeons have lost the ability to use direct visualization and the sense of touch to make determinations as to the presence of blood vessels in the surgical field. Consequently, surgeons must make the determination whether blood vessels are present in the surgical field based primarily on convention and experience. Unfortunately, anatomical irregularities frequently occur because of congenital anomalies, scarring from prior surgeries, and body habitus (e.g., obesity). Systems and methods that would permit surgeons to determine the presence and/or the characteristics of vessels in the surgical field during surgery (potentially in real time or near real time) under such conditions would be a significant advantage.

On the other hand, while it would be advantageous to include systems and methods that provide information regarding the presence of blood vessels in the surgical field, the adoption of such systems and methods would be impeded if these systems and methods were to complicate the manufacture and/or use of the associated surgical instruments. This is particularly true in the field of minimally-invasive surgery, where surgical instrument design involves a complex balance of competing interests, and space for the integration of new technologies is at a considerable premium.

As set forth in more detail below, the present disclosure describes a system and method for electrically coupling a surgical system or part thereof embodying advantageous alternatives to the existing systems and methods, which may provide for improved identification for avoidance or isolation of the vessel without undue complication of the surgical instrument.

### Summary

A surgical system according to the present invention is defined in appended independent claim 1.

Preferred embodiments are defined in the appended dependent claims.

A system according to the preamble of claim 1 is described in document WO2004030527 A1.

### Brief Description of the Drawings

The disclosure will be more fully understood from the following description taken in conjunction with the accompanying drawings. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. None of the drawings is necessarily to scale.
Fig. 1 is a schematic diagram of a surgical system according to an embodiment of the present disclosure;
Fig. 2 is an enlarged, fragmentary view of a transmittance-based embodiment of the surgical instrument of Fig. 1 with light emitter and light sensors, and a section of an vessel illustrated as disposed between the light emitter and light sensors;
Fig. 3 is an enlarged, fragmentary view of a reflectance-based embodiment of the surgical instrument of Fig. 1 with light emitter and light sensor in fixed relation to each other, and a section of a vessel illustrated as proximate the light emitter and light sensor;
Fig. 4 is an enlarged, fragmentary view of a reflectance-based embodiment of the surgical instrument of Fig. 1 with light emitter and light sensor in moveable or adjustable relation to each other, and a section of a vessel illustrated as proximate the light emitter and light sensor;
Fig. 5 is a perspective view of a surgical system according to an embodiment wherein a tubular shaft used to electrically couple a light emitter and a light sensor to the remainder of the system is part of the surgical instrument;
Fig. 6 is a perspective view of a surgical system according to an embodiment wherein a tubular shaft used to electrically couple a light emitter and a light sensor to the remainder of the system is separate from the surgical instrument;
Fig. 7 is a perspective view of a surgical system according to an alternative embodiment wherein a tubular shaft used to electrically couple a light emitter and a light sensor to the remainder of the system is separate from the surgical instrument;
Fig. 8 is a cross-sectional view of a tubular shaft with a discontinuity about its periphery;
Fig. 9 is a cross-sectional view of a first embodiment wherein one or more conductors are disposed on an outer surface of a wall of the tubular shaft;
Fig. 10 is a cross-sectional view of a second embodiment, not part of the present invention, wherein one or more conductors are disposed on an outer surface of a wall of the tubular shaft;
Fig. 11 is a cross-sectional view of a third embodiment wherein one or more conductors are disposed on an outer surface of a wall of the tubular shaft;
Fig. 12 is a cross-sectional view of a fourth embodiment, not part of the present invention, wherein one or more conductors are disposed on an outer surface of a wall of the tubular shaft;
Fig. 13 is a cross-sectional view of a first embodiment, not part of the present invention, wherein one or more conductors define an inner surface of a wall of the tubular shaft;
Fig. 14 is a cross-sectional view of a second embodiment, not part of the present invention, wherein one or more conductors define an inner surface of a wall of the tubular shaft;
Fig. 15 is a perspective view of an embodiment, not part of the present invention, wherein one or more conductors define a wall of the tubular shaft;
Fig. 16 is a perspective view of an alternative embodiment, not part of the present invention, wherein one or more conductors define a wall of the tubular shaft; and
Fig. 17 is a perspective view of a connector that may be used with one or more of the embodiments disclosed above.

### Detailed Description of Various Embodiments

A surgical system according to an embodiment of the present disclosure includes at least one light emitter, at least one light sensor, and a controller. The system may also include a surgical instrument as well.

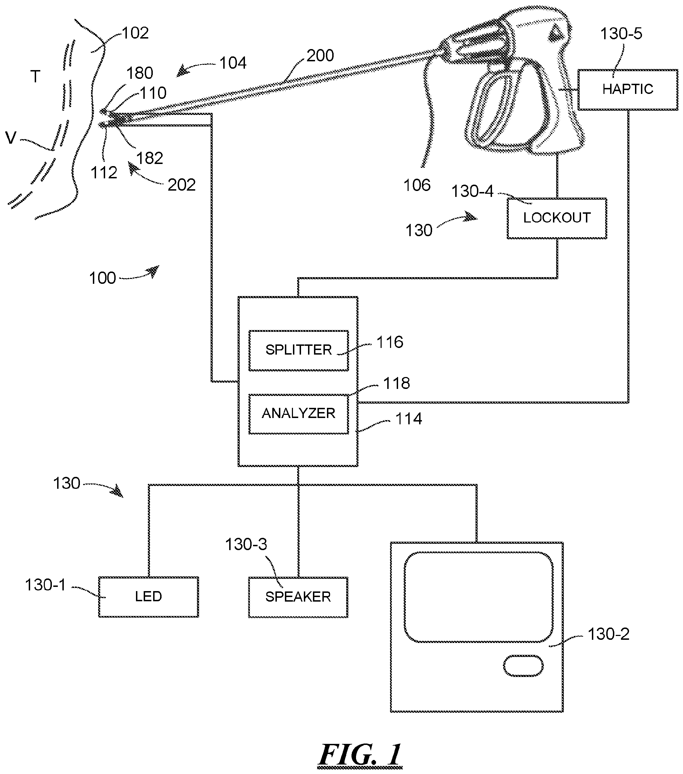

Figs. 1-4 illustrate embodiments of such a surgical system 100 used to determine, for example, the presence and/or a spatial characteristic (e.g., diameter) of a vessel, V, within a region 102 of tissue, T, proximate to a working end 104 of a surgical instrument 106. It will be understood that the vessel V may be connected to other vessels with the region 102 of tissue T, and in addition, the vessel V may extend beyond the region 102 so as to be in fluid communication with other organs (e.g., the heart) also found in the body of the patient. Furthermore, while the tissue T appears in Figs. 1-4 to surround fully the vessel V (in terms of both circumference and length) to a particular depth, this need not be the case in all instances where the system 100 is used. For example, the tissue T may only partially surround the circumference of and/or only surround a section of the length of the vessel V, or the tissue T may overlie the vessel V in a very thin layer. As further nonlimiting examples, the vessel V may be a blood vessel, and the tissue T may be connective tissue, adipose tissue and/or liver tissue.

The surgical system 100 includes at least one light emitter 110 (or simply the light emitter 110), at least one light sensor or detector 112 (or simply the light sensor 112), and a controller 114 coupled to the light emitter 110 and the light sensor 112. As noted above, the system 100 also may include the surgical instrument 106.

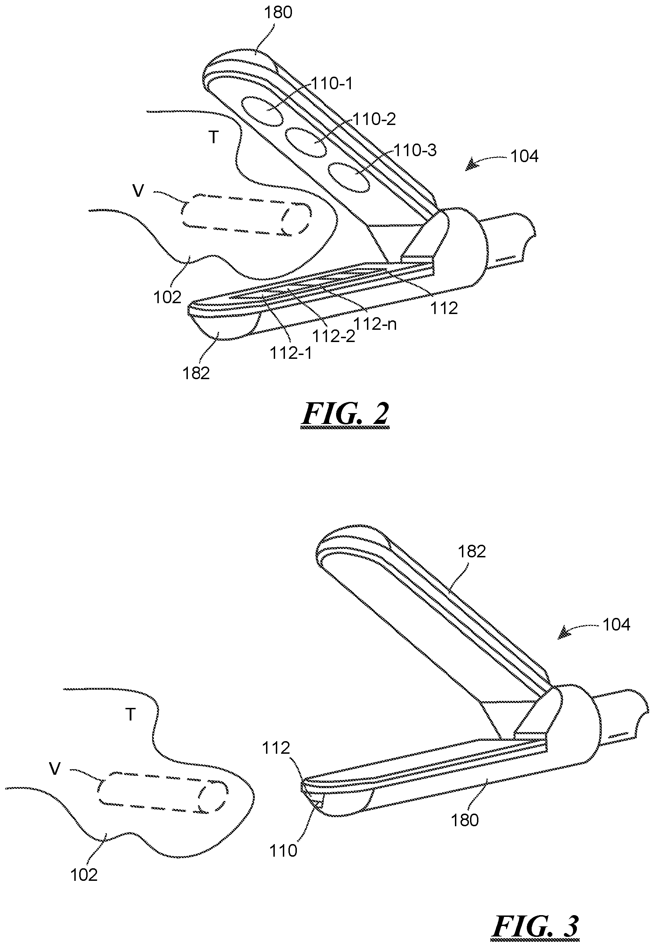

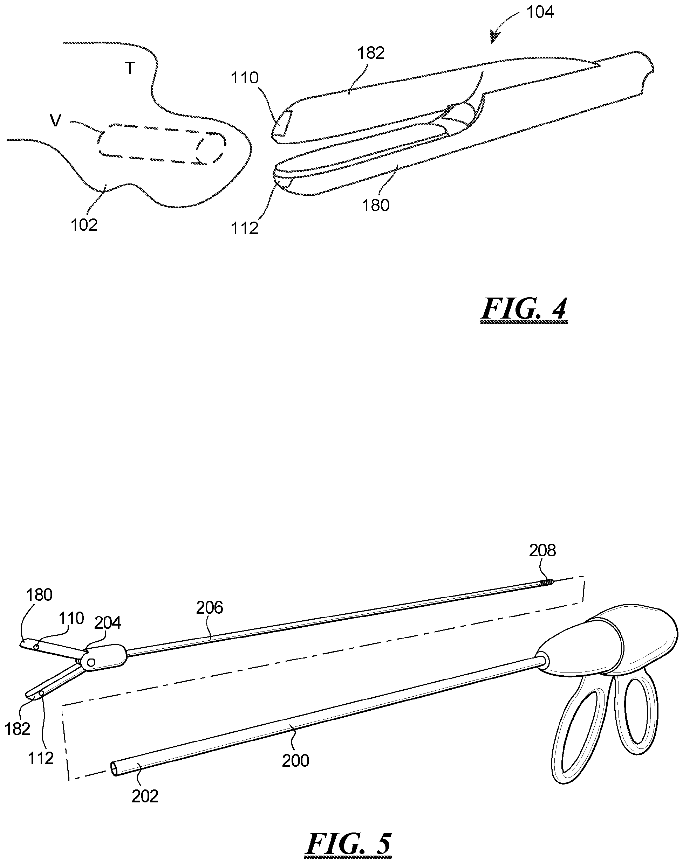

The light emitter 110 is disposed at the working end 104 of the surgical instrument 106. The light sensor 112 is also disposed at the working end 104 of the surgical instrument 106. As illustrated in Fig. 2, the system 100 may operate according to a transmittance-based approach, such that the light sensor 112 is disposed opposite the light emitter 110, for example on opposite jaws of a surgical instrument 106. As illustrated in Fig. 3, the system 100 may operate according to a reflectance-based approach, such that the light emitter 110 and the light sensor 112 may be disposed in fixed relation to each other, for example on a single jaw of a two-jaw device, such as a thermal ligation device, or on a blunt end of a laparoscopic dissection tool. As illustrated in Fig. 4, even with the system 100 operating according to a reflectance-based approach, the light emitter 110 and the light sensor 112 may be disposed so that the spacing between the light emitter 110 and the light sensor 112 may be adjusted, for example by positioning the light emitter 110 at the end or tip of one of the jaws of a two-jaw device and the light sensor 112 at the end or tip of the other the jaws of the two-jaw device.

The light emitter 110 is adapted to emit light of at least one wavelength. For example, the light emitter 110 may emit light having a wavelength of 660 nm. This may be achieved with a single element, or a plurality of elements (which elements may be arranged or configured into an array, for example, as explained in detail below). In a similar fashion, the light sensor 112 is adapted to detect light at the at least one wavelength (e.g., 660 nm). According to the embodiments described herein, the light sensor 112 includes a plurality of elements, which elements are arranged or configured into an array.

According to certain embodiments, the light emitter 110 may be configured to emit light of at least two different wavelengths, and the light sensor 112 may be configured to detect light at the at least two different wavelengths. As one example, the light emitter 110 may emit and the light sensor 112 may detect light in the visible range and light in the near-infrared or infrared range. Specifically, the light emitter 110 may emit and the light sensor 112 may detect light at 660 nm and at 910 nm. Such an embodiment may be used, for example, to ensure optimal penetration of blood vessel V and the surrounding tissue T under *in vivo* conditions.

Depending upon the effect of changes in blood flow, light of a third wavelength may also be emitted and sensed. That is, if the method of detection is found to be sensitive to varying rates of blood flow in the vessel of interest, light at 810 nm (*i.e*., at the isobestic point) may be emitted and sensed to permit normalization of the results to limit or eliminate the effects of changes in blood flow rate.

According to certain embodiments of this disclosure, the individual light sensor 112 is adapted to generate a signal comprising a first pulsatile component and a second non-pulsatile component. It will be recognized that the first pulsatile component may be an alternating current (AC) component of the signal, while the second non-pulsatile component may be a direct current (DC) component. Where the light sensor 112 is in the form of an array, the pulsatile and non-pulsatile information may be generated for each element of the array, or at least for each element of the array that defines the at least one row of the array.

As to the pulsatile component, it will be recognized that a blood vessel may be described as having a characteristic pulsation of approximately 60 pulses (or beats) per minute. While this may vary with the patient's age and condition, the range of pulsation is typically between 60 and 100 pulses (or beats) per minute. The light sensor 112 will produce a signal (that is passed to the controller 114) with a particular AC waveform that corresponds to the movement of the blood through the vessel. In particular, the AC waveform corresponds to the light absorption by the pulsatile blood flow within the vessel. On the other hand, the DC component corresponds principally to light absorption and scattering by the surrounding tissues.

According to such embodiments, the controller 114 is coupled to the light sensor 112, and thus may include a splitter 116 to separate the first pulsatile component from the second non-pulsatile component for each element of the light sensor array 112. The controller 114 may also include an analyzer 118 to determine the presence of the and/or characteristic(s) of the vessel V within the region 102 proximate to the working end 104 of the surgical instrument 106 based (at least in part) on the pulsatile component. To display, indicate or otherwise convey the size of the vessel V within the region 102, the controller 114 may be coupled to an output device or indicator 130 (see Fig. 1), which may provide a visible, audible, tactile or other signal to the user of the instrument 106.

According to certain embodiments, the splitter 116 and the analyzer 118 may be defined by one or more electrical circuit components. According to other embodiments, one or more processors (or simply, the processor) may be programmed to perform the actions of the splitter 116 and the analyzer 118. According to still further embodiments, the splitter 116 and the analyzer 118 may be defined in part by electrical circuit components and in part by a processor programmed to perform the actions of the splitter 116 and the analyzer 118.

For example, the splitter 116 may include or be defined by the processor programmed to separate the first pulsatile component from the second non-pulsatile component. Further, the analyzer 118 may include or be defined by the processor programmed to determine the presence of (or to quantify the size of, for example) the vessel V within the region 102 proximate to the working end 104 of the surgical instrument 106 based on the first pulsatile component. The instructions by which the processor is programmed may be stored on a memory associated with the processor, which memory may include one or more tangible non-transitory computer readable memories, having computer executable instructions stored thereon, which when executed by the processor, may cause the one or more processors to carry out one or more actions.

While the foregoing general discussion of the system 100 made reference to the light emitter 110 and the light sensor 112, as recognized above, the actual specifics of the implementation of the light emitter 100 and light sensor 112 may include more than a single element or component.

For example, referring to the embodiment of Fig. 2, the light emitter 110 may include one or more elements. According to an embodiment schematically illustrated in Fig. 2, the light sensor 112 may include a first light emitter 110-1, a second light emitter 110-2, and a third light emitter 110-3. All of the light emitters may be adapted to emit light at a particular wavelength (e.g., 660 nm), or certain emitters may emit light at different wavelengths than other emitters. Each light emitter may be a light emitting diode, for example.

As to those embodiments wherein the light emitter 110 is in the form of and array including one or more light emitting diodes, as is illustrated in Fig. 2 for example, the diodes may be arranged in the form of a one-dimensional, two-dimensional or three-dimensional array. An example of a one-dimensional array may include disposing the diodes along a line in a single plane, while an example of a two-dimensional array may include disposing the diodes in a plurality of rows and columns in a single plane. Further example of a two-dimensional array may include disposing the diodes along a line on or in a curved surface. A three-dimensional array may include diodes disposed in more than one plane, such as in a plurality of rows and columns on or in a curved surface.

The light sensor 112 also may include one or more elements. According to an embodiment illustrated in Fig. 2, the light sensor 112 may include a first light sensor 112-1, a second light sensor 112-2, an n-th light sensor 112-n, and so on. As was the case with the light emitters 110-1, 110-2, 110-3, the light sensors 112-1, 112-2, 112-3 may be arranged in an array, and the discussion in regard to the arrays above applied with equal force here.

In fact, where the array of light sensors 112 includes a row of light sensors (such as in Fig. 2), the array 112 may be referred to in the alternative as a linear array. The individual light sensors of the array 112 may be disposed adjacent each other, or the light sensors may be spaced from each other. It may even be possible for the individual light sensors that define a row of light sensors to be separated from each other by light sensors that define a different row or column of the array. According to a particular embodiment, however, the array may comprise a charge coupled device (CCD), and in particular linear CCD imaging device comprising a plurality of pixels. As a further alternative, a CMOS sensor array may be used.

While the arrangement of the light emitter 110 and the light sensor 112 may vary relative to the reflectance-based embodiments of Figs. 3 and 4, it is equally true that the light emitter 110 and the light sensor 112 may involve a plurality of elements.

Contrasting the arraignment illustrated in Fig. 3 with that of Fig. 2, the light emitter 110 and light sensor 112 are disposed generally facing in a common direction (i.e., the direction of the tissue sample of interest). This does not require the emitter 110 and the sensor 112 to be generally disposed in a common plane, although this is preferred. According to certain embodiments, the emitter 110 and sensor 112 may be formed integrally (i.e., as one piece) with jaws 180, 182 of a surgical instrument 106 (see Figs. 2 and 3), although other options are possible, as discussed below. In this manner, light emitted by the emitter 110 and reflected by the tissue of interest may be captured by the light sensor 112.

Further, it is believed that the spacing between the emitter 110 and the sensor 112 may influence the light received by the sensor 112. For example, it has been determined that a spacing between the emitter 110 and the sensor 112 of 5 mm may permit detection of vessels from 0 mm to 12 mm from the surface of the tissue. It is believed that increasing the spacing between the emitter 110 and the sensor 112 may permit the light to penetrate even deeper into the tissue, permitting vessel detection at even greater depths.

On the other hand, the emitter 110 and sensor 112 may be disposed so as to be mounted in a fixed relationship to each other, or a moveable or adjustable relationship. In particular, Fig. 3 illustrates an embodiment wherein emitter 110 and sensor 112 are at a fixed spacing relative to each other, in that they are both mounted in a first jaw 180 of the instrument 106. Such an embodiment would permit the user to be confident that the vessels detected are within 12 mm from the working end 104 of the instrument 106. By contrast, the embodiment of Fig. 4 has the sensor 112 mounted in a first jaw 180 of the instrument 106 and the emitter 110 mounted in a second jaw 182 of the tool 106. Such an embodiment would permit the user to vary the depth of detection simply by varying the distance between the jaws 180, 182 of the instrument 106: with the jaws 180, 182 closed, the user may probe for shallow vessels (i.e., vessels disposed within 12 mm of the tissue surface), while with the jaws 180, 182 open, the user may probe for deeper vessels (i.e., vessels disposed greater than 12 mm below the tissue surface). According to the embodiment illustrated in Fig. 4, the control structure for operating the jaws 180, 182 may include a mechanism for modifying the distance between the jaws 180, 182 in a controlled fashion (e.g., in discrete increments) so that the user can determine the jaw spacing (and thus the detection depth) without visualization of the jaws 180, 182.

As mentioned above, the light emitter 110 of either Fig. 3 or 4 may include one or more elements. According to such an embodiment, all of the elements may be adapted to emit light at a particular wavelength (e.g., 660 nm), or certain elements may emit light at different wavelengths than other elements.

As to those embodiments wherein the light emitter 110 is in the form of an array including one or more light emitting diodes, the diodes may be arranged in the form of a one-dimensional, two-dimensional or three-dimensional array. An example of a one-dimensional array may include disposing the diodes along a line in a single plane, while an example of a two-dimensional array may include disposing the diodes in a plurality of rows and columns in a single plane. Further example of a two-dimensional array may include disposing the diodes along a line on or in a curved surface. A three-dimensional array may include diodes disposed in more than one plane, such as in a plurality of rows and columns on or in a curved surface.

The light sensor 112 according to the embodiments of Figs. 3 and 4 also may include one or more individual elements. As was the case with the light emitter 110, the elements of the light sensor 112 may be arranged in an array, and the discussion about the arrays above applied with equal force here.

In addition, the system 100 may include hardware and software in addition to the emitter 110, sensor 112, and controller 114. For example, where more than one emitter 110 is used, a drive controller may be provided to control the switching of the individual emitter elements. In a similar fashion, a multiplexer may be provided where more than one sensor 112 is included, which multiplexer may be coupled to the sensors 112 and to an amplifier. Further, the controller 114 may include filters and analog-to-digital conversion as may be required.

As a consequence, there are a considerable number of electrical connections that need to be made between the components that define the light emitter 110 and the light sensor 112 and the other electrical components of the system 100. Considering that the light emitter 110 and the light sensor 112 are located at the working end 104 of the surgical instrument 106, while the controller 114 and the other components of the system 100 are generally located at a distance to the working end 104 of the instrument 106, these electrical connections will need to extend over that distance as well. This is particularly true relative to minimally-invasive surgery, where the working end 104 of the surgical instrument 106 is disposed at a distal end of an elongated member, while the controller 104 is typically located no closer than the proximal end of the elongated member 204. The elongated member may be in the form of a tubular shaft through which, for example, the actuation mechanism responsible for the opening and closing of the jaws 180, 182 is passed from the proximal end to the distal end of the shaft.

As mentioned above, complicating the instrument 106 through the incorporation of the system 100 may well impede adoption of the technology. Consequently, a system that permits electrical coupling between the light emitter 110/light sensor 112 and the controller 114 without requiring redesign or repackaging of the operating mechanisms, such as the actuation mechanism, of the surgical instrument 106 would be preferable. Moreover, it is desirable if such a solution does not require the inclusion of additional lumens in the elongated member or shaft, because the inclusion of such additional lumens would disturb the arrangement of the existing structures within the shaft. Furthermore, the creation of an elongated member, such as a tubular shaft or a rod, with lumens that extend from the distal end to a proximal end with sufficient precision to permit a wire or other conductor to be disposed along the lumen may present a difficult, if not impossible, manufacturing problem. Consequently, a number of embodiments for electrical coupling the light emitter 110/light sensor 112 and the remainder of the electrical components are proposed that may enhance the adoptability of such technology.

In general terms, the surgical system 100 according to these embodiments includes a tubular shaft having a wall defining an outer surface and an inner surface disposed about an inner space, the tubular shaft having a proximal end and a distal end. The system 100 also includes the aforementioned light emitter 110 and light sensor 112 disposed at the distal end of the tubular shaft. Further, the system 100 includes one or more conductors electrically coupled to the light emitter or the light sensor, the one or more conductors extending from the distal end of the tubular shaft to the proximal end of the shaft, The one or more conductors disposed radially outward of the inner surface of the tubular shaft.

In doing so, the structure of the surgical system 100 maintains the inner space free of any equipment required by the surgical system 100 to determine the presence and/or characteristics of, for example, blood vessels in the surgical field. As such, the technology may be adopted without altering the design of a surgical instrument that is also part of the surgical instrument. Moreover, certain embodiments disclosed below may also permit the electrical coupling between the light emitter 110/light sensor 112 and the remainder of the electrical components to occur without altering either the inner or the outer diameter of a shaft that is part of the surgical instrument. Furthermore, this is done without introducing lumens into the wall of the tubular shaft that might weaken the shaft or complicate its manufacturability.

Such a surgical system may provide similar advantages even if the detection modality is not an optical system, such that the system 100 includes, for example, a sensor disposed at the distal end of the tubular shaft.

As illustrated in Fig. 1, the tubular shaft 200 may be part of the surgical instrument 106. According to such an embodiment, the light emitter 110 and the light sensor 112 may be disposed at the distal end 202 of the tubular shaft 200, for example, as a consequence of first and second jaws 180, 182 being attached to the distal end 202 of the tubular shaft 200 and the light emitter 110 and the light sensor 112 being disposed on at least one of the first and second jaws 180, 182. In fact, as illustrated, the light emitter 110 is attached to the first jaw 180, while the light sensor 112 is attached to the second jaw 182.

An alternative embodiment is illustrated in Fig. 5. According to this embodiment, the light emitter 110 and the light sensor 112 are attached to a distal end 204 of an elongated structure 206, and the elongated structure 206 (which may also be a tubular rod or shaft) is disposed within the tubular shaft 200 so that the light emitter 110 and light sensor 112 are disposed at the distal end 202 of the tubular shaft 200. In particular, first and second jaws 180, 182 are attached to the distal end 204 of the elongated structure 206, and the light emitter 110 and the light sensor 112 are disposed on at least one of the first and second jaws 180, 182. For example, the light emitter 110 may be attached to the first jaw 180 and the light sensor 112 may be attached to the second jaw 182.

The disposition of the light emitter 110 and light sensor 112 at the distal end 202 of the shaft 200 may be achieved, for example, as a consequence of the relative lengths of the shaft 200 and the shaft 206.

The light emitter 110 and the light sensor 112 may be fixedly attached to the remainder of the surgical instrument 106 so that the emitter 110 and sensor 112 are fixedly disposed at the distal end 202 of the shaft 200 by providing a connector (such as an external threading) at a proximal end 208 of the shaft 206 and providing a mating connector (e.g., a recess with an internal threading) at a proximal end of the shaft 200. While providing a fixed attachment (under operational conditions), the connectors may permit the shaft 206 and associated equipment to be removable (and thus disposable separate from the remainder of the instrument 106) or permanently attached. This connection may also mechanically couple the jaws 180, 182 to the remainder of the actuation mechanism.

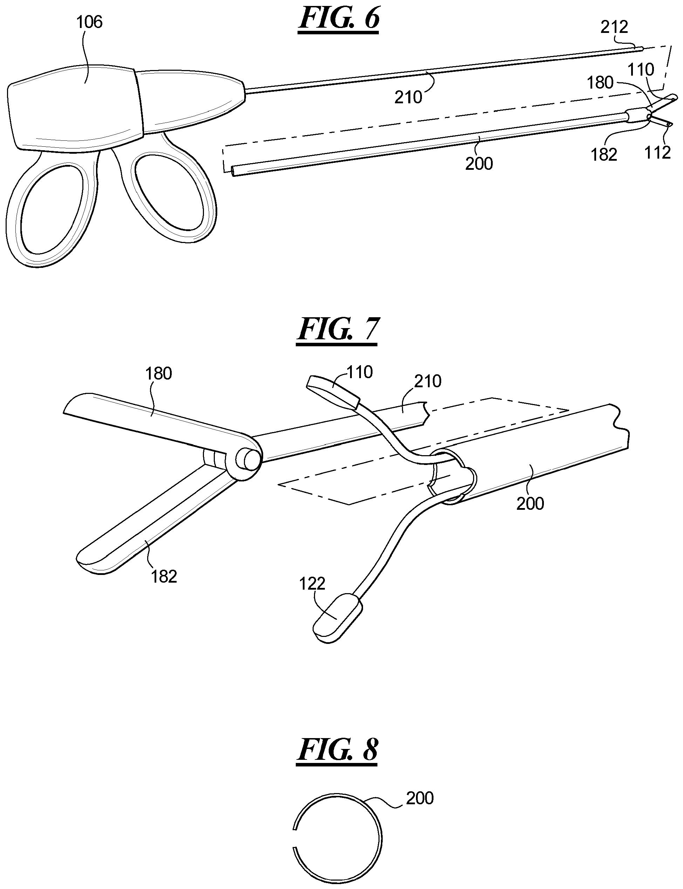

It is also possible for the tubular shaft 200 to be a structure that is separate from the surgical instrument 106. For example, Fig. 6 illustrates an embodiment of the system 100 wherein the tubular shaft 200 is disposed about an elongated element 210 (which may be a tubular shaft or rod) of a surgical instrument 106. The embodiment of Fig. 6 may be considered to be a reversal of parts relative to the embodiment of Fig. 5, with the jaws 180, 182 attached to the distal end 202 of the shaft 200, and the elongated element 210 disposed within the tubular shaft 200. Further, the elongated element 210 may have a connector disposed at a distal end 212 (such as an external threading) which mates with a connector disposed at the distal end 200 (such as a recess with an internal threading) of the shaft 200. This connection may also mechanically couple the jaws 180, 182 to the remainder of the actuation mechanism.

Fig. 7 illustrates a further embodiment of the system 100 wherein the tubular shaft 200 is disposed about the elongated element 210 (which may be a tubular shaft) of a surgical instrument 106. According to this embodiment, the instrument 106 includes first and second jaws 180, 182 attached to the distal end of the elongated element 210, the light emitter 110 and the light sensor 112 may be disposed on at least one of the first and second jaws 180, 182. As illustrated, the light emitter 110 is intended to be attached to the first jaw 180, and the light sensor 112 is intended to be attached to the second jaw 182.

In particular, the shaft 200 is intended to be disposed about the elongated shaft 210 by passing the shaft 210 through the interior of the shaft 200. Where the tubular shaft 200 has a continuous cross-section, this may require that the shaft 200 be installed about the shaft 210 before the jaws 180, 182 are attached to the shaft 210. However, if the tubular shaft 200 has a discontinuity (such as a split) in its cross-section, the shaft 200 may be partially deformed to permit the shaft 210 to be disposed within the shaft 200, and then the shaft 200 may be allowed to resume its original shape (for example, as a natural consequence of the material used to manufacture the shaft 200).

The discontinuity may be of such a limited nature that the shaft 200 appears to be continuous in its original shape or after it has been disposed about the shaft 210. Alternatively, the discontinuity may be of a more extreme nature, such that the shaft 200 retains its tubular, hollow appearance, but appears less like a continuous annulus or ring-shape in cross-section and more like a c-shape, for example (compare Figs. 7 and 8). It will be recognized that the exact nature of the shape may vary, is not restricted to either an exactly circularly-shaped annulus or rounded c-shape as illustrated.

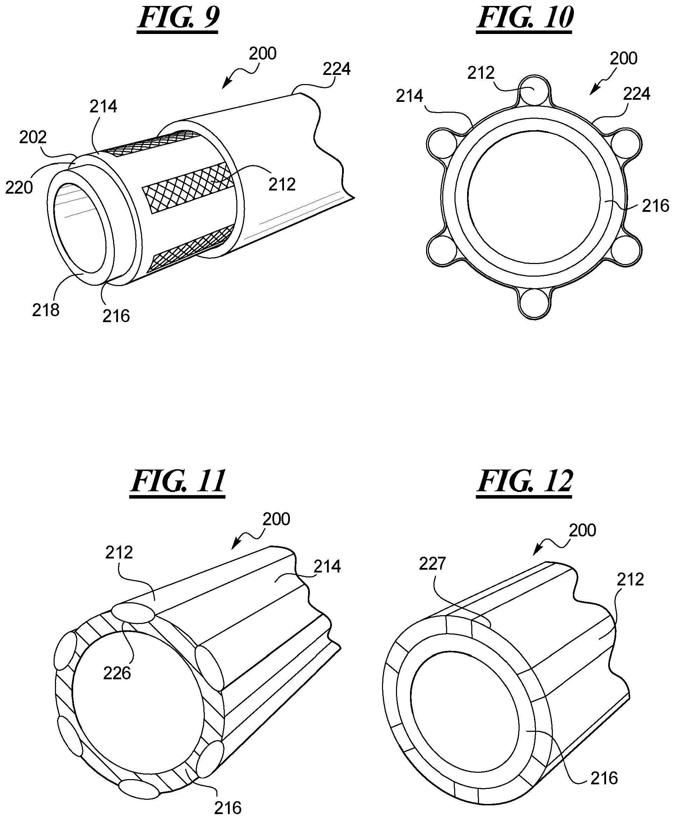

As discussed above, according to the embodiments of the system 100, the tubular shaft 200 has a wall that defines an outer surface and an inner surface disposed about an inner space, the tubular shaft having a proximal end and a distal end. Further, the system 100 includes one or more conductors electrically coupled to the light emitter or the light sensor, the one or more conductors extending from the distal end of the tubular shaft to the proximal end of the shaft, the one or more conductors disposed radially outward of the inner surface of the tubular shaft. To better illustrate these concepts, reference is now made to the embodiments illustrated in Figs. 9-15, any of which may be combined with the features of the embodiments illustrated in Figs. 1 and 5-8 as discussed immediately above. The embodiments illustrated in figures 10 and 12-15 are not part of the present invention.

In the embodiments of Figs. 9-12, the one or more conductors 212 are disposed on an outer surface 214 of a wall 216 of the tubular shaft 200.

As illustrated in Fig. 9, the wall 216 includes a tubular structural element 218 disposed radially inward of a tubular insulating element 220, the outer surface of the tubular insulating element 220 defining the outer surface 214 of the wall 216. The tubular structural element 218 provides a certain degree of rigidity to the tubular shaft, such the shaft does not kink or otherwise interfere with mechanisms disposed within the shaft. However, the degree of rigidity is relative, such that according to certain embodiments, the shaft 200 may be sufficiently flexible to be able to deflect between its ends without kinking, for example. According to one embodiment, the tubular structural element 218 may be a metal tube, and the insulating element 220 may be a ceramic coating disposed on the metal tube. However, the insulating element 220 need not be merely a coating, but may instead be defined by a separate tube of insulating material 220 that is disposed outside and joined to an outer surface of the structural element 218. The insulating element 220 may even add to or provide rigidity to the tubular shaft 200. Furthermore, where the tubular structural element 218 is not made of an electrically conductive material, it may not be necessary to provide an insulating element 220 between the tubular structural element 218 and the one or more conductors 212.

According to the present invention, as illustrated in Fig. 9, the one or more conductors 212 are disposed on the outer surface 214 by printing traces of electrically conductive material (e.g., copper) on the outer surface 214. To protect this material along at least part of the distance between the distal end 202 and a proximal end of the tubular shaft 200, a layer of protective material 224 may disposed over the one or more conductors 212 and the outer surface 214 of the wall 216. This layer 224 may be a coating or a wrap, and may be at least partially insulating as well. The layer 224 may be removed to expose the one or more conductors 212 to electrically couple the one or more conductors 212 to the light emitter 110 and the light sensor 112, and to other equipment as well (e.g., the controller 114).

As illustrated in Fig. 10, the one or more conductors 212 include one or more wires. A layer of protective material 224 may be disposed over the conductors 212 and the outer surface 214 of the wall. As the wires 212 may themselves be at least partially surrounded by a material that acts at least partially as an insulator, it may not be necessary to provide a separate layer of insulating material according to such an embodiment.

As illustrated in Fig. 11, the wall 216 may have one or more grooves 226 formed in the outer surface 214, the one or more conductors 212 disposed in the one or more grooves.226. As illustrated, the wall 216 has one groove 226 for each conductor 212. While the conductors 212 illustrated in Fig. 11 are in the form of wires and therefore not covered by the present invention, the conductors 212 may be in the form of printed traces according to one alternative embodiment covered by the present invention. Such an embodiment may or may not include a layer of insulating material and may or may not include a layer disposed over the one or more conductors and the outer surface of the wall, depending upon the characteristics of the conductors 212 used. In the alternative to the wall 216 having one or more grooves 226, one or more spacers 227 may be disposed between the one or more conductors 212 as in Fig. 12, which spacers 227 may physically space the conductors 212 as well as electrically insulate the conductors 212 from each other.

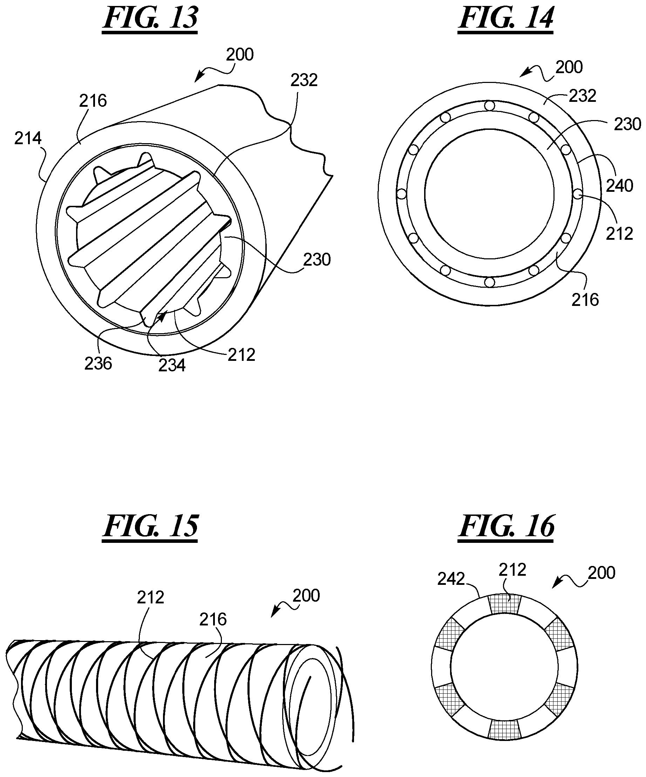

Instead of being disposed on an outer surface 214 of the wall 216 of the tubular shaft 210, according to embodiments not part of the present invention, the one or more conductors 212 may be disposed in the wall 216, for example to define an inner surface of the wall 216 of the tubular shaft 200, as in the embodiments of Figs. 13 and 14.

As illustrated in Fig. 13, the wall 216 comprises a tubular insulating element 230 disposed radially inwardly of a tubular structural element 232, and the one or more conductors 212 are disposed radially inward of the tubular insulating element 230. In fact, in the illustrated embodiment, the wall 216 comprises a layer of electrically conductive material (e.g., copper) 234 disposed radially inwardly of the tubular insulating element 230, the layer of electrically conductive material 234 having discontinuities 236 that extend from the distal end 202 to the proximal end of the shaft 200 to separate the layer of electrically conductive material 234 into the one or more conductors 212. The discontinuities 236 may be formed after the layer of electrically conductive material 234 is disposed on the tubular insulating element 230 by moving a die through an inner space defined by the inner surface, the die having edges that remove the electrically conductive material (and potentially at least part of the insulating element 230) so as to form the discontinuities 236 in the layer 234.

In the alternative, as illustrated in Fig. 14, the one or more conductors 212 may be formed on the tubular insulating element 230, and the tubular insulating element 230 is attached to an inner surface 240 of the tubular structural element 232. According to such an embodiment, the conductors 212 may be printed on the tubular insulating element 230 before the element 230 is formed into its tubular shape. For example, the insulating element 230 may be initially flat, the conductors 212 (in the form of traces) may be printed onto the insulating element 230, then the insulating element may be formed into a tubular shape and attached to the inner surface 240 of the structural element 232.

As a still further alternative not part of the present invention, the one or more conductors 212 may be formed within the wall 216. As illustrated in Fig. 15, the conductors 212 in the form of one or more wires may be set within a matrix to define a composite that defines the wall 216, the wires providing a structural element for the composite as well as providing an electrical coupling between the light emitter 110/light sensor 212 and the remainder of the electrical equipment. Similar to the embodiment of Fig. 12, spacers 242 may be added to the wall 216 to separate the conductors 212 physically and electrically, as well as to provide a structural element, as illustrated in Fig. 16.

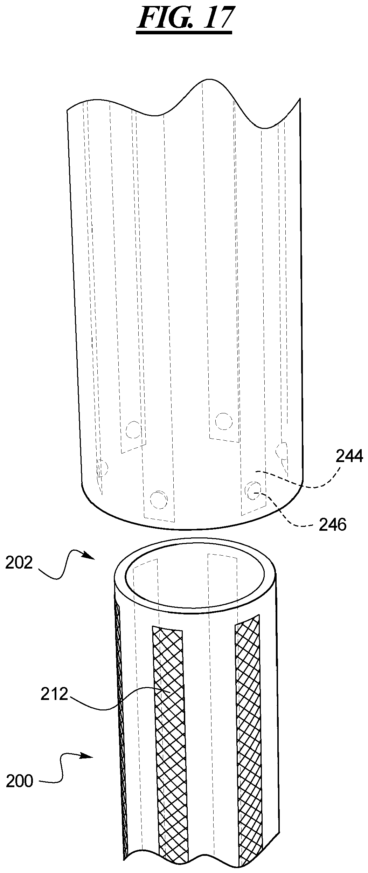

The conductors 212 and the light emitter 110/light sensor 112, as well as the conductors 212 and the remainder of the electrical components, may be directly coupled by joining the conductors to the leads of the light emitter 110, light sensor 112, or other components. According to embodiments not part of the present invention, this joining may be permanent, in that a tool or considerable force may be required to break or sever the connection, and that the connection will require repair to reform the connection. An example of such a connection may be a soldered junction. Alternatively, where a removable connection is desired (such as may be desired for the embodiments illustrated in Figs. 5-7), a plug and socket pair, not part of the present invention, may be used at the end where the connection is to be formed. A further alternative according to the present invention is illustrated in Fig. 17, for use with an embodiment similar to that illustrated in Fig. 9. The tubular shaft 200 on which the one or more conductors 212 are disposed (e.g., by disposing the leads on an insulating surface) is fitted into a socket 244 having a plurality of spring contacts 246; as illustrated, at least one contact 244 is provided for each conductor 212. This arrangement provides the potential for the shaft 200 to be fitted into the socket and releasably connected to the remainder of the surgical instrument 106, and the electrical coupling to occur.

As mentioned above, the system 100 may include other equipment as well. For example, relative to the indicator 130 used in conjunction with controller 114, a variety of output devices have been illustrated in Fig. 1. A light emitting diode 130-1 may be attached to or incorporated into the associated surgical instrument 106, and may even be disposed at the working end 104 of the instrument 106. Alternatively or in addition, an alert may be displayed on a video monitor 130-2 being used for the surgery, or may cause an image on the monitor to change color or to flash, change size or otherwise change appearance. The indicator 130 may also be in the form of or include a speaker 130-3 that provides an auditory alarm. The indicator 130 also may be in the form of or may incorporate a safety lockout 130-4 associated with the surgical instrument 106 that interrupts use of the instrument 106. For example, the lockout could prevent ligation or cauterization where the surgical instrument 106 is a thermal ligature device. As a still further example, the indicator 130 also may be in the form of a haptic feedback system, such as a vibrator 130-5, which may be attached to or formed integral with a handle or handpiece of the surgical instrument 106 to provide a tactile indication or alarm. Various combinations of these particular forms of the indicator 130 may also be used.

As mentioned above, the surgical system 100 may also include the surgical instrument 106 with the working end 104, to which the light emitter 110 and light sensor 112 are attached (in the alternative, removably/reversibly or permanently/irreversibly). The light emitter 110 and the light sensor 112 may instead be formed integrally (i.e., as one piece) with the surgical instrument 106. It is further possible that the light emitter and light sensor be attached to a separate instrument or tool that is used in conjunction with the surgical instrument or tool 106.

As noted above, the surgical instrument 106 may be a thermal ligature device in one embodiment. In another embodiment, the surgical instrument 106 may simply be a grasper or grasping forceps having opposing jaws. According to still further embodiments, the surgical instrument may be other surgical instruments such as surgical staplers, clip appliers, and robotic surgical systems, for example. According to still other embodiments, the surgical instrument may have no other function that to carry the light emitters/light sensors and to place them within a surgical field. The illustration of a single embodiment is not intended to preclude the use of the system 100 with other surgical instruments or tools 106.

In conclusion, although the preceding text sets forth a detailed description of different embodiments of the invention, it should be understood that the legal scope of the invention is defined by the words of the claims set forth at the end of this patent. The detailed description is to be construed as exemplary only and does not describe every possible embodiment of the invention since describing every possible embodiment would be impractical, if not impossible. Numerous alternative embodiments could be implemented, using either current technology or technology developed after the filing date of this patent, which would still fall within the scope of the claims defining the invention.

It should also be understood that, unless a term is expressly defined in this patent using the sentence "As used herein, the term '_____' is hereby defined to mean..." or a similar sentence, there is no intent to limit the meaning of that term, either expressly or by implication, beyond its plain or ordinary meaning, and such term should not be interpreted to be limited in scope based on any statement made in any section of this patent (other than the language of the claims). To the extent that any term recited in the claims at the end of this patent is referred to in this patent in a manner consistent with a single meaning, that is done for sake of clarity only so as to not confuse the reader, and it is not intended that such claim term be limited, by implication or otherwise, to that single meaning.

## Claims

1. A surgical system comprising:
a tubular shaft (200) having a wall (216) defining an outer surface (214) and an inner surface disposed about an inner space, the tubular shaft (200) having a proximal end and a distal end (202);
a light emitter (110) and a light sensor (112) disposed at the distal end (202) of the tubular shaft (200); and
one or more conductors (212) electrically coupled to the light emitter (110) or the light sensor (112), the one or more conductors (212) extending from the distal end (202) of the tubular shaft (200) to the proximal end of the shaft (200),
wherein the one or more conductors (212) are disposed radially outward of the inner surface of the tubular shaft,
**characterized in that**:
the one or more conductors (212) consist of printed traces disposed on an outer surface (214) of the wall (216) of the tubular shaft (200), and
the tubular shaft (200) is fitted into a socket (244) having a plurality of spring contacts (246), at least one contact (246) is provided for each conductor (212), and the tubular shaft (200) is releasably connected thereby to the remainder of the surgical system.

2. The surgical system according to claim 1, wherein the wall (216) comprises a tubular structural element (218) disposed radially inward of a tubular insulating element (220), the outer surface of the tubular insulating element (220) defining the outer surface (214) of the wall (216).

3. The surgical system according to claim 2, wherein the tubular structural element (218) comprises a metal tube and the insulating element (220) comprises a ceramic coating disposed on the metal tube.

4. The surgical system according to any one of claims 1-3, wherein the wall has a circular cross-section.

5. The surgical system according to any one of claims 1-4, further comprising a layer (224) disposed over the one or more conductors and the outer surface of the wall, and the layer is one of a coating and a wrap.

6. The surgical system according to any one of claims 1-5, wherein the tubular shaft (200) defines part of a surgical instrument (106), and the light emitter (110) and the light sensor (112) are attached to a distal end (204) of an elongated structure (206), the elongated structure (206) disposed within the tubular shaft (200).

7. The surgical system according to claim 6, further comprising first and second jaws (180, 182) attached to the distal end (204) of the elongated structure (206), the light emitter (110) and the light sensor (112) disposed on at least one of the first and second jaws (180, 182).

8. The surgical system according to claim 7, wherein the light emitter (110) is attached to the first jaw (180) and the light sensor (112) is attached to the second jaw (182).

9. The surgical system according to any one of claims 6-8, wherein the elongated structure (206) comprises a shaft.

10. The surgical system according to any one of claims 1-5, further comprising a surgical instrument (106), the tubular shaft (200) disposed about an elongated element (210) of the surgical instrument (106), the elongated element (210) comprising a shaft of the surgical instrument (106).

11. The surgical system according to claim 10, wherein the surgical instrument (106) comprises first and second jaws (180, 182) attached to the distal end (202) of the elongated structure (210), the light emitter (110) and the light sensor (112) disposed on at least one of the first and second jaws (180, 182).

12. The surgical system according to claim 10, wherein the light emitter (110) is attached to the first jaw (180) and the light sensor (112) is attached to the second jaw (182).

13. The surgical system according to any one of claims 10-12, wherein the tubular shaft (200) has a continuous cross-section.

14. The surgical system according to any one of claims 10-12, wherein the tubular shaft (200) has a discontinuity in its cross-section.

15. The surgical system according to claim 14, wherein the tubular shaft (200) has a c-shape in cross-section.

## Patentansprüche

1. Chirurgisches System, umfassend:
einen rohrförmigen Schaft (200), der eine Wand (216) aufweist, die eine Außenfläche (214) und eine Innenfläche, die um einen Innenraum angeordnet ist, definiert, wobei der rohrförmige Schaft (200) ein proximales Ende und ein distales Ende (202) aufweist,
einen Lichtsender (110) und einen Lichtsensor (112), die am distalen Ende (202) des rohrförmigen Schafts (200) angeordnet sind, und
einen oder mehrere Leiter (212), die elektrisch mit dem Lichtsender (110) oder dem Lichtsensor (112) gekoppelt sind, wobei der eine oder die mehreren Leiter (212) sich vom distalen Ende (202) des rohrförmigen Schafts (200) zum proximalen Ende des Schafts (200) erstrecken,
wobei der eine oder die mehreren Leiter (212) radial nach außen von der Innenfläche des rohrförmigen Schafts angeordnet sind,
**dadurch gekennzeichnet, dass**:
der eine oder die mehreren Leiter (212) aus gedruckten Bahnen bestehen, die auf einer Außenfläche (214) der Wand (216) des rohrförmigen Schafts (200) angeordnet sind, und
der rohrförmige Schaft (200) in eine Buchse (244) mit einer Vielzahl von Federkontakten (246) eingesetzt ist, mindestens ein Kontakt (246) für jeden Leiter (212) bereitgestellt ist und der rohrförmige Schaft (200) dadurch lösbar mit dem Rest des chirurgischen Systems verbunden ist.

2. Chirurgisches System nach Anspruch 1, wobei die Wand (216) ein rohrförmiges Strukturelement (218) umfasst, das radial nach innen von einem rohrförmigen Isolierelement (220) angeordnet ist, wobei die Außenfläche des rohrförmigen Isolierelements (220) die Außenfläche (214) der Wand (216) definiert.

3. Chirurgisches System nach Anspruch 2, wobei das rohrförmige Strukturelement (218) ein Metallrohr umfasst und das Isolierelement (220) eine Keramikbeschichtung umfasst, die auf dem Metallrohr angeordnet ist.

4. Chirurgisches System nach einem der Ansprüche 1 bis 3, wobei die Wand einen kreisförmigen Querschnitt aufweist.

5. Chirurgisches System nach einem der Ansprüche 1 bis 4, ferner umfassend eine Schicht (224), die über dem einen oder den mehreren Leitern und der Außenfläche der Wand angeordnet ist, und die Schicht ist eine aus einer Beschichtung und einer Umhüllung.

6. Chirurgisches System nach einem der Ansprüche 1 bis 5, wobei der rohrförmige Schaft (200) einen Teil eines chirurgischen Instruments (106) definiert und der Lichtsender (110) und der Lichtsensor (112) an einem distalen Ende (204) einer länglichen Struktur (206) angebracht sind, wobei die längliche Struktur (206) innerhalb des rohrförmigen Schafts (200) angeordnet ist.

7. Chirurgisches System nach Anspruch 6, ferner umfassend eine erste und eine zweite Backe (180, 182), die am distalen Ende (204) der länglichen Struktur (206) angebracht sind, wobei der Lichtsender (110) und der Lichtsensor (112) an mindestens einer der ersten und der zweiten Backe (180, 182) angeordnet sind.

8. Chirurgisches System nach Anspruch 7, wobei der Lichtsender (110) an der ersten Backe (180) angebracht ist und der Lichtsensor (112) an der zweiten Backe (182) angebracht ist.

9. Chirurgisches System nach einem der Ansprüche 6 bis 8, wobei die längliche Struktur (206) einen Schaft umfasst.

10. Chirurgisches System nach einem der Ansprüche 1 bis 5, ferner umfassend ein chirurgisches Instrument (106), wobei der rohrförmige Schaft (200) um ein längliches Element (210) des chirurgischen Instruments (106) angeordnet ist, wobei das längliche Element (210) einen Schaft des chirurgischen Instruments (106) umfasst.

11. Chirurgisches System nach Anspruch 10, wobei das chirurgische Instrument (106) eine erste und eine zweite Backe (180, 182) umfasst, die am distalen Ende (202) der länglichen Struktur (210) angebracht sind, wobei der Lichtsender (110) und der Lichtsensor (112) an mindestens einer der ersten und der zweiten Backe (180, 182) angeordnet sind.

12. Chirurgisches System nach Anspruch 10, wobei der Lichtsender (110) an der ersten Backe (180) angebracht ist und der Lichtsensor (112) an der zweiten Backe (182) angebracht ist.

13. Chirurgisches System nach einem der Ansprüche 10 bis 12, wobei der rohrförmige Schaft (200) einen durchgehenden Querschnitt aufweist.

14. Chirurgisches System nach einem der Ansprüche 10 bis 12, wobei der rohrförmige Schaft (200) eine Unterbrechung in seinem Querschnitt aufweist.

15. Chirurgisches System nach Anspruch 14, wobei der rohrförmige Schaft (200) einen c-förmigen Querschnitt aufweist.

## Revendications

1. Système chirurgical comprenant :
une tige tubulaire (200) ayant une paroi (216) définissant une surface externe (214) et une surface interne disposée autour d'un espace interne, la tige tubulaire (200) ayant une extrémité proximale et une extrémité distale (202) ;
un émetteur de lumière (110) et un capteur de lumière (112) disposés à l'extrémité distale (202) de la tige tubulaire (200) ; et
un ou plusieurs conducteurs (212) couplés électriquement à l'émetteur de lumière (110) ou au capteur de lumière (112), l'un ou plusieurs conducteurs (212) s'étendant à partir de l'extrémité distale (202) de la tige tubulaire (200) à la l'extrémité proximale de la tige (200),
dans lequel l'un ou plusieurs conducteurs (212) sont disposés radialement vers l'extérieur de la surface intérieure de la tige tubulaire,
**caractérisé en ce que** :
l'un ou plusieurs conducteurs (212) sont constitués de traces imprimées disposées sur une surface extérieure (214) de la paroi (216) de la tige tubulaire (200), et
la tige tubulaire (200) est montée dans une douille (244) ayant une pluralité de contacts à ressort (246), au moins un contact (246) est prévu pour chaque conducteur (212), et la tige tubulaire (200) est ainsi reliée de manière amovible au reste du système chirurgical.

2. Système chirurgical selon la revendication 1, dans lequel la paroi (216) comprend un élément structurel tubulaire (218) disposé radialement vers l'intérieur d'un élément tubulaire isolant (220), la surface extérieure de l'élément tubulaire isolant (220) définissant la surface extérieure (214) de la paroi (216).

3. Système chirurgical selon la revendication 2, dans lequel l'élément structurel tubulaire (218) comprend un tube métallique et l'élément isolant (220) comprend un revêtement céramique disposé sur le tube métallique.

4. Système chirurgical selon l'une quelconque des revendications 1 à 3, dans lequel la paroi a une section transversale circulaire.

5. Système chirurgical selon l'une quelconque des revendications 1 à 4, comprenant en outre une couche (224) disposée sur l'un ou plusieurs conducteurs et la surface extérieure de la paroi, et la couche est l'une d'un revêtement et d'une enveloppe.

6. Système chirurgical selon l'une quelconque des revendications 1 à 5, dans lequel la tige tubulaire (200) définit une partie d'un instrument chirurgical (106), et l'émetteur de lumière (110) et le capteur de lumière (112) sont fixés à une extrémité distale (204) d'une structure allongée (206), la structure allongée (206) étant disposée à l'intérieur de la tige tubulaire (200).

7. Système chirurgical selon la revendication 6, comprenant en outre des première et deuxième mâchoires (180, 182) fixées à l'extrémité distale (204) de la structure allongée (206), l'émetteur de lumière (110) et le capteur de lumière (112) étant disposés sur au moins l'une des première et deuxième mâchoires (180, 182).

8. Système chirurgical selon la revendication 7, dans lequel l'émetteur de lumière (110) est fixé à la première mâchoire (180) et le capteur de lumière (112) est fixé à la deuxième mâchoire (182).

9. Système chirurgical selon l'une quelconque des revendications 6 à 8, dans lequel la structure allongée (206) comprend une tige.

10. Système chirurgical selon l'une quelconque des revendications 1 à 5, comprenant en outre un instrument chirurgical (106), la tige tubulaire (200) étant disposée autour d'un élément allongé (210) de l'instrument chirurgical (106), l'élément allongé (210) comprenant une tige de l'instrument chirurgical (106).

11. Système chirurgical selon la revendication 10, dans lequel l'instrument chirurgical (106) comprend des première et deuxième mâchoires (180, 182) fixées à l'extrémité distale (202) de la structure allongée (210), l'émetteur de lumière (110) et le capteur de lumière (112) étant disposés sur au moins l'une des première et deuxième mâchoires (180, 182).

12. Système chirurgical selon la revendication 10, dans lequel l'émetteur de lumière (110) est fixé à la première mâchoire (180) et le capteur de lumière (112) est fixé à la deuxième mâchoire (182).

13. Système chirurgical selon l'une quelconque des revendications 10 à 12, dans lequel la tige tubulaire (200) a une section transversale continue.

14. Système chirurgical selon l'une quelconque des revendications 10 à 12, dans lequel la tige tubulaire (200) a une discontinuité dans sa section transversale.

15. Système chirurgical selon la revendication 14, dans lequel la tige tubulaire (200) a une section transversale en forme de C.
